Europäisches Patentamt

European Patent Office

Office européen des brevets

(11) Publication number: **0 288 179 B1**

(12) **EUROPEAN PATENT SPECIFICATION**

(45) Date of publication of patent specification: **08.07.92**

(51) Int. Cl.⁵: **G01N 33/577**, G01N 33/70, G01N 33/541, G01N 33/543, C12Q 1/50, C12N 5/00, C12P 21/00

(21) Application number: **88303056.1**

(22) Date of filing: **06.04.88**

(54) **CKMB assay, monoclonal antibodies for use therein and hybrid cell lines.**

(30) Priority: **09.04.87 US 36057**

(43) Date of publication of application:
**26.10.88 Bulletin 88/43**

(45) Publication of the grant of the patent:
**08.07.92 Bulletin 92/28**

(84) Designated Contracting States:
**AT BE CH DE ES FR GB IT LI NL SE**

(56) References cited:
**WO-A-87/03094**

**CHEMICAL ABSTRACTS, vol. 95, no. 15, 12 October 1981, Columbus, OH (US); WUERZ-BURG et al, p. 234, no. 128032r**

**CHEMICAL ABSTRACTS, vol. 107, no. 17, 26 October 1987, Columbus, OH (US); HAFKEN-SCHEID et al., p. 298, no. 149805b**

**CHEMICAL ABSTRACTS, vol. 104, no. 21, 26 May 1986, Columbus, OH (US); RUELLAND et al., p. 252, no. 182114m**

**CLINICAL CHEMISTRY, vol. 32, no. 10, 1986; V.V.MURTHY et al., pp. 1956-1959**

**CHEMICAL ABSTRACTS, vol. 102, no. 13, 01 April 1985, Columbus, OH (US); JACKSON et al, p. 323, no. 108923s**

(73) Proprietor: **Ciba Corning Diagnostics Corp.**
**63 North Street**
**Medfield Massachusetts 02052(US)**

(72) Inventor: **Piran, Uri**
**16 Woodland Street**
**Sharon, MA 02062(US)**
Inventor: **Vitkauskas, Christine Anne**
**11 Howard Avenue**
**Foxboro, MA 02035(US)**

(74) Representative: **Froud, Clive et al**
**Elkington and Fife Prospect House 8 Pembroke Road**
**Sevenoaks, Kent TN13 1XR(GB)**

## Description

This invention relates to a CKMB assay, to monoclonal antibodies for use therein and to hybrid cell lines.

The enzyme creatine kinase (i.e., ATP:creatine N-phosophotransferase, EC 2.7.3.2) is a dimer composed of either two M monomers (CKMM), two B monomers (CKBB), or one M and one B monomer (CKMB). Each of these isoenzymes has the same molecular weight and catalyzes the same reaction, i.e., the reversible phosphorylation of creatine. They differ in their molecular structure and charge in an alkaline buffer, thus allowing them to be separated by electrophoresis. See Lott, J.A., Creatine Kinase, Lactate Dehydrogenase and their Isoenzymes in Clinical Medicine, Corning Medical Publication, Medfield, Massachusetts, 1984; and Lang, H. (ed), Creatine Kinase Isoenzymes, Pathophysiology and Clinical Application, Springer-Verlag, New York, 1981.

The correct diagnosis of acute myocardial infarction (MI) is a critical clinical challenge which can determine the survival of a patient. Since CKMB is present almost exclusively in cardiac muscle, serum concentrations of CKMB can be used to determine if damage (e.g., MI) to the cardiac muscle has occurred. In comparison with other enzymes, such as, lactate dehydrogenase and aspartate transaminase, CKMB is a more specific marker of MI because it is more localized in cardiac muscle. See Galen, R. S., et al., "Isoenzymes of CPK and LDH in Myocardial Infarction and Certain Other Diseases", Pathobiol. Annu., 5:283-315, (1975); and Lott, J. A., et al., "Serum Enzymes and Isoenzymes in the Diagnosis and Differential Diagnosis of Myocardial Infarction", Clin. Chem., 26:1241, (1980). Furthermore, leakage of CKMB from an infarct precedes that of the other enzymes, thus providing the opportunity for earlier diagnosis. See Kiyasu, J. Y., "Current Status of Detecting CK-MB for Patient Management", Amer. Clin. Prod., 4:23-31 (1985). In addition to indicating the presence or absence of an infarct, serum concentrations of CKMB can also be used to assess the extent of MI and to determine if reinfarction has occurred. See Lott, J. A., supra; Konttinen, A., "The Isoenzymes of Creatine Kinase in Various Diseases", in Enzymes in Health and Disease, Inaug. Scient. Meet. Int. Soc. Clin. Enzymol., London, 1977, pages 149-153; Kupper N. and Bliefeld W., "Serum Enzyme changes in Patients with Cardiac Disease", in Advances in Clinical Enzymology, 1979, pages 106-122; and Hackshaw B., Clinical Chemistry, 30:1285 (1984).

In view of its diagnostic importance, numerous techniques have been developed for measuring CKMB concentrations in serum. See Abbott et al., "Methods for CK-MB Analysis Used to Monitor Cardiac Patients", Journal of Clinical Immunoassay, 8:147-151 (1985); Chan et al., "Immunoenzymetric Assay for Creatine Kinase MB with Subunit-Specific Monoclonal Antibodies Compared with an Immunochemical Method and Electrophoresis", Clinical Chemistry, 31:465-469 (1985); Jackson et al., "Two-Site Monoclonal Antibody Assays for Human Heart-and Brain-Type Creatine Kinase", Clinical Chemistry, 30:1157-1162 (1984); and PCT patent publication No. WO 86/00992, published February 13, 1986. Unfortunately, the available assay methodologies have had various drawbacks such that the full diagnostic potential of CKMB has to date not been realized.

For example, among the techniques which have been developed to measure the concentration of CKMB in serum are electrophoretic or ion exchange separation of CK fractions followed by the determination of the CK activity in the MB fraction. Radioimmunoassays and immunoinhibition assays, including two-site immunometric assays and immunoinhibition/immunoprecipitation assays, have also been developed.

All of these approaches can give false positives due to artifacts and variants that migrate similarly to CKMB (separation type techniques) or to interferences, such as those caused by CKBB, CKMM, macro CK-1, and other enzymes (immuno-techniques). In addition, electrophoresis and two-site immunometric assays involving multiple incubation/wash steps are lengthy and tedious.

Previous two-site immunometric assays for CKMB have been based on sandwich formation between anti-CKMM and anti-CKBB antibodies. See PCT patent publication No. WO 86/00992, published February 13, 1986; Youens, J. E. B., et al., "Clinical and Analytical Validation of an Enzymometric Assay for Creatine Kinase-MB Isoenzyme", Ann. Clin. Biochem., 23:463-469, (1986); Medeiros, L. J., et al., "Quantification of CK-MB by the Quick MB and Tandem-E CK-MB Immunoassays: Comparison to Electrophoresis", J. Clin. Immunoassay, 8:152-156, (1985); and Chan. D. W., et al., "Immunoenzymetric Assay for Creatine Kinase-MB with Subunit-Specific Monoclonal Antibodies Compared with an Immunochemical Method and Electrophoresis", Clin. Chem., 31:465-469, (1985). As demonstrated below, this approach, when used in a single incubation assay, results in standard curves which are susceptible to interference by CKMM and CKBB.

Recently, the use of an anti-CKMB monoclonal antibody in a CKMB assay has been reported. See Vaidya et al., "Direct Measurement of Creatine Kinase-MB Activity in Serum after Extraction with a Monoclonal Antibody Specific to the MB Isoenzyme", Clinical Chemistry, 32:657-663 (1986). The monoclonal antibody employed in this study was a mouse antibody of the IgG2b type. The antibody was coupled to polymer beads, and the beads were used to extract CKMB from serum samples.The amount of CKMB extracted was measured enzymatically by incubating the beads with CK reagent for 30 minutes at 37°C, centrifuging the beads, removing a portion of the supernatant, and measuring the absorbance of that portion at 340 nm.

The Vaidya et al. assay suffers from a number of problems. In the first place, because the assay measures CKMB activity, rather than mass concentration, it is subject to inaccuracies due to deterioration in CKMB activity between the time a blood sample is taken and the time the assay is performed. As is well known, such time periods, as well as the environmental conditions to which different samples are exposed during such time periods, can vary widely from sample to sample in a clinical setting. The mass concentration of CKMB in a sample, on the other hand, is relatively insensitive to the particular storage times and conditions to which the sample has been exposed. See Murthy et al., "Activity Concentration and Mass Concentration (Monoclonal Antibody Immunoenzyometric Method) Compared for Creatine Kinase MB Isoenzyme in Serum", Clin. Chem., 32:1956 (1986).

In addition to the foregoing, the monoclonal antibody disclosed in the Vaidya et al. report is not particularly well-suited for use in a commercial assay. Specifically, as indicated above, the Vaidya et al. antibody is of the IgG2b type. As is known in the art, antibodies of this type are sensitive to freezing, tend to precipitate in solutions having low salt concentrations, and are more susceptible to degradation by proteolytic enzymes than other types of IgG antibodies, specifically, IgG1 type antibodies. Also, in comparison with IgG2b type antibodies, IgG1 type antibodies are usually easier to derivatize, i.e., easier to label with an enzyme, chemiluminescent material, or the like. See Goding, J. W., Monoclonal Antibodies: Principles and Practice, Academic Press, Inc., New York, New York, 1983, page 15; and Parham, P., "On the Fragmentation of Monoclonal IgG1, IgG2a, and IgG2b from BALB/c Mice", Journal of Immunology, 131:2895-2902 (1983).

In view of the foregoing, it is an object of the present invention to provide improved assays for measuring the concentrations of CKMB in biological fluids, in particular, serum samples. More specifically, it is an object of the invention to provide improved CKMB assays which are easy to use, have high precision, and are not subject to substantial interferences from CKMM, CKBB, macro CK-1, or other enzymes found in serum samples.

In connection with these objects, it is a further object of the invention to provide anti-CKMB monoclonal antibodies which are well-suited for use in commercial CKMB assays. Specifically, it is an object of the invention to provide mouse anti-CKMB monoclonal antibodies of the IgG1 type.

To achieve the foregoing and other objects, the invention provides a method for detecting CKMB in a biological fluid comprising the steps of:

(a) forming a mixture of (i) a sample of the biological fluid, (ii) an antibody to CKB which has been bound to a solid support, and (iii) a labelled, monoclonal antibody to CKMB;

(b) incubating the mixture;

(c) separating the solid support from the mixture; and

(d) detecting the amount of label associated with the solid support.

In certain preferred embodiments of the invention the solid support comprises magnetic particles and the anti-CKMB monoclonal antibody is a mouse monoclonal antibody of the IgG1 type which has been labelled with a chemiluminescent material.

In addition to the foregoing method, the invention also provides anti-CKMB monoclonal antibodies which are produced by murine-derived hybrid cell lines and which are of the IgG1 type. Such monoclonal antibodies can be used in the practice of the above preferred method for detecting CKMB or in other methods now known or subsequently developed for detecting this or other enzymes.

More particularly, the present invention provides a method for detecting the MB isoenzyme of creatine kinase in a biological fluid characterised in that it comprises:

(a) forming a mixture of (i) a sample of the biological fluid, (ii) an antibody to the B monomer of creatine kinase, the said antibody being bound to a solid support, and (iii) a labelled, mouse monoclonal antibody of the IgG1 type specific to the MB isoenzyme of creatine kinase;

(b) incubating the mixture;

(c) separating the solid support from the mixture; and

(d) detecting the amount of label associated with the solid support.

(a) - (d) being completed in about 1 hour.

The present invention also provides a monoclonal antibody characterised in that it is produced by a murine-derived hybrid cell line, the said antibody being capable of specifically binding to at least one antigenic determinant of the MB isoenzyme of human creatine kinase and being of the IgG1 type and not being substantially cross-reactive with the BB and MM isoenzymes of human creative kinase.

The present invention further provides a murine-derived hybrid cell line ATCC HB 9389 or HB 9388.

Referring to the accompanying illustrative drawings:

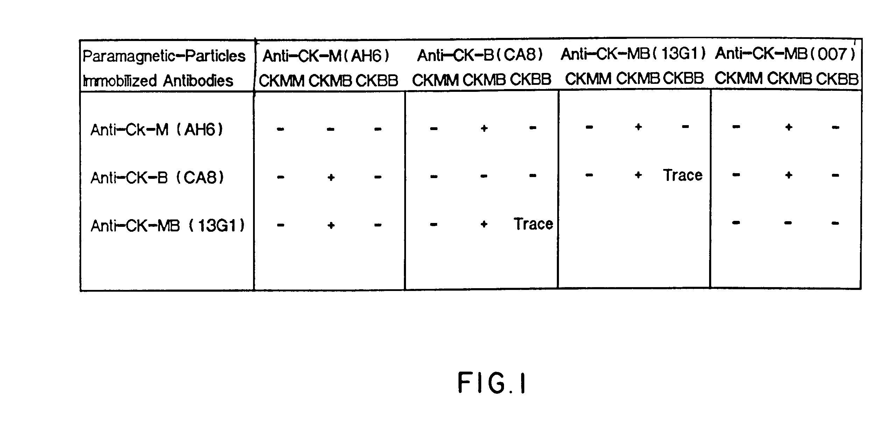

Figure 1 is a table illustrating the specificity and "sandwich" compatibility of the following anti-CK antibodies; AH6 -- anti-CKM; CA8 -- anti-CKB; 13G1 -- anti-CKMB; and 007 -- anti-CKMB. A standard curve (0-1000 ug/l) was constructed for each of CKBB, CKMM, and CKMB using the capture/tracer antibodies listed in the table. Compatible combinations (" + " combinations) had a sensitivity of at least 1 ug/l. Incompatible combinations ("-" combinations) had less than 0.1% immunoreactivity. The 13G1 antibody was found to have a "trace" cross reactivity with CKBB (about 1%).

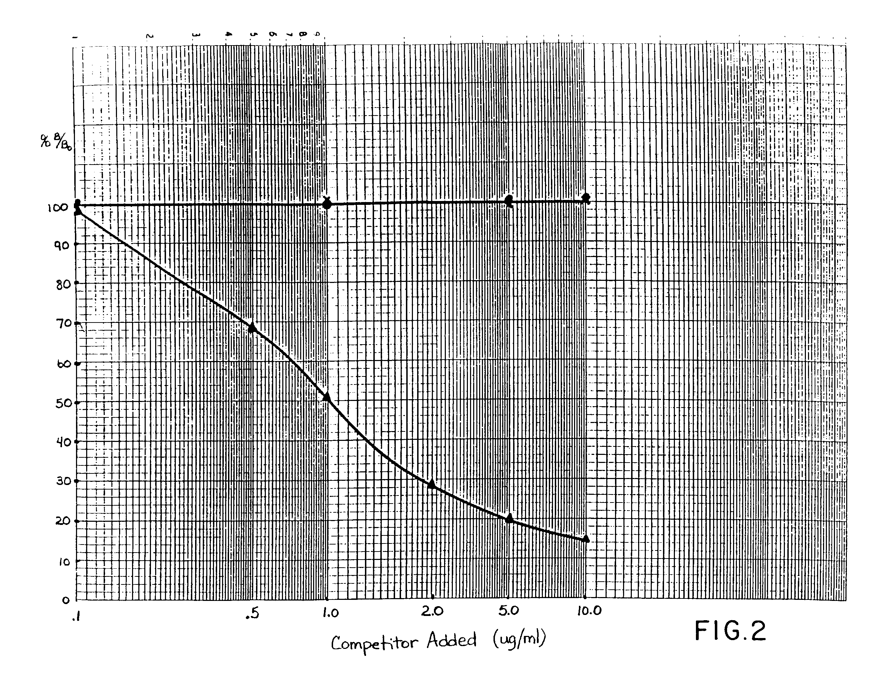

Figure 2 illustrates the specificity of the 007 antibody. In particular, this figure shows the binding of labelled CKMB to the 007 antibody in the presence of various concentrations of purified CKMB (triangles), CKMM (crosses), and CKBB (circles). The data is plotted as the percent of counts bound in the presence of the competitor compared to the counts for the 0 standard ($B_0$) versus the concentration of competitor added (ug/ml).

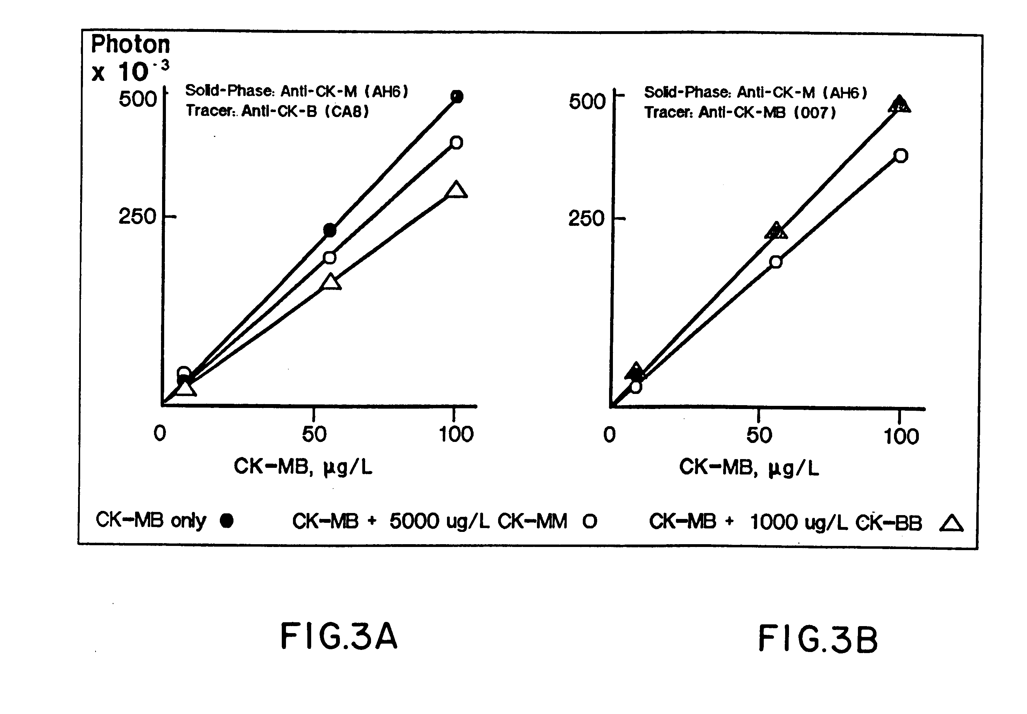

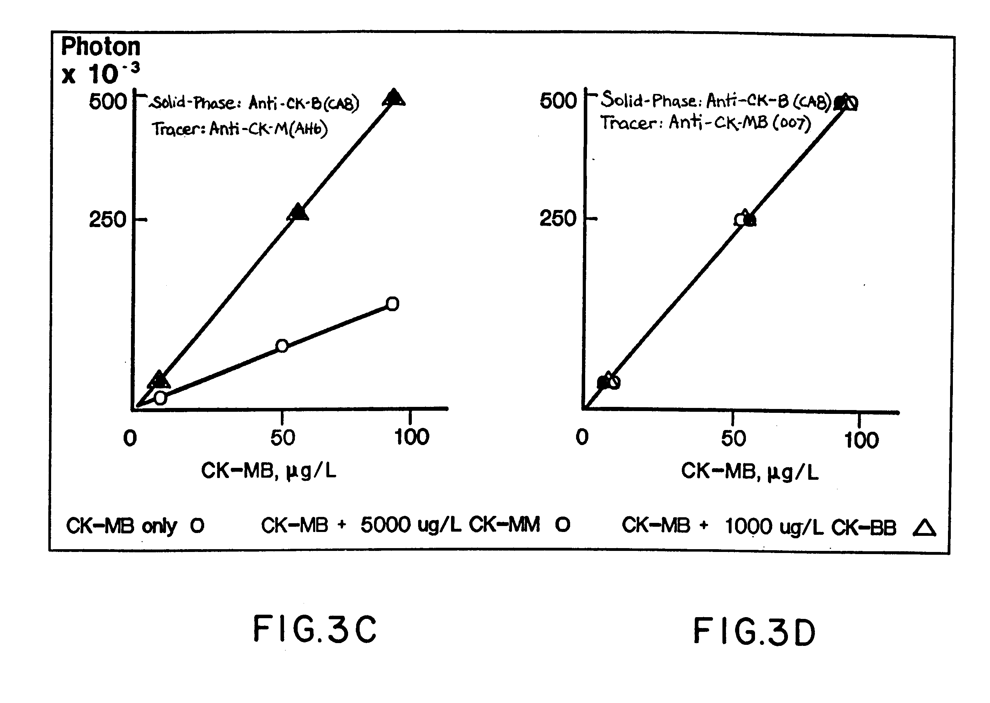

Figure 3 illustrates the susceptibility of CKMB standard curves to inhibition by CKMM and CKBB in various assay configurations. The closed circles represent standards containing only CKMB, the open circles represent standards to which 5000 ug/l of CKMM has been added; and the triangles represent standards to which 1000 ug/l of CKBB has been added.

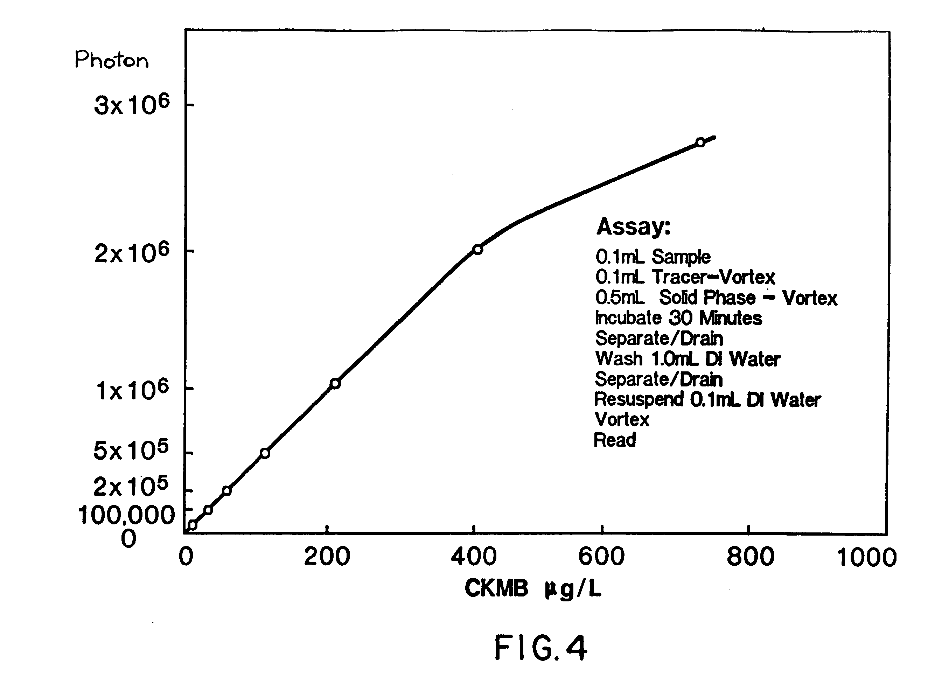

Figure 4 illustrates a typical standard curve achieved through the use of an anti-CKB antibody (CA8) as the capture antibody and an anti-CKMB antibody (007) as the tracer antibody.

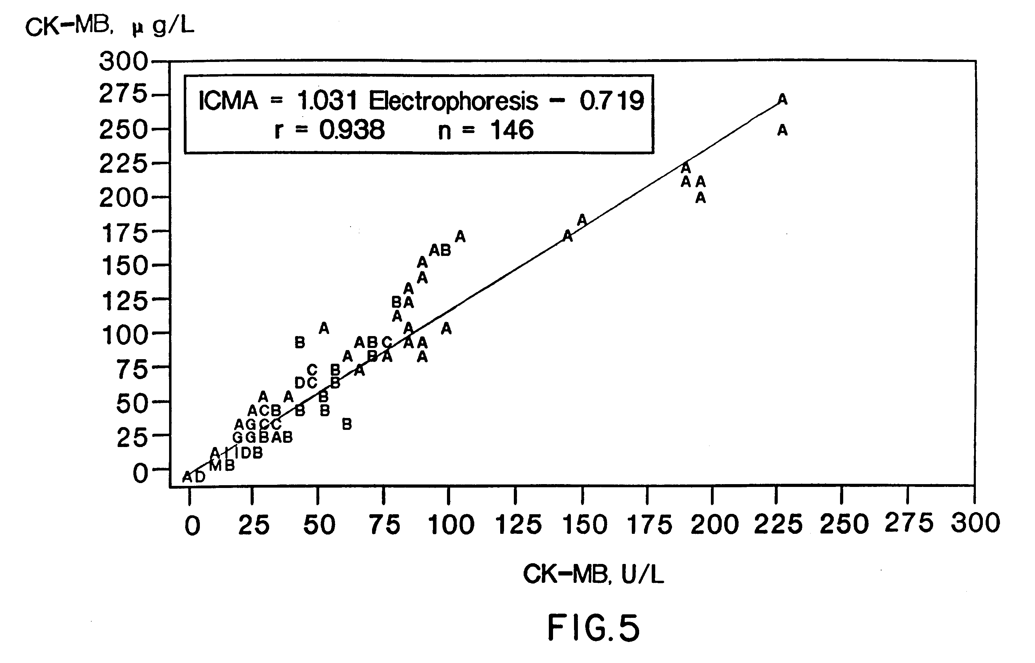

Figure 5 illustrates the correlation between a CKMB assay performed in accordance with the present invention (anti-CKB (CA8) was the capture antibody and anti-CKMB (007) was the tracer antibody), and an assay performed in accordance with a conventional electrophoretic separation technique. Patient sera were tested in duplicates in both methods. The alphabetical data points shown in Figure 5 represent the number of sample results at a particular coordinate as follows: A = 1, B = 2, C = 3, D = 4, G = 7, I = 9 and M = 13.

As discussed above, the present invention relates to a two-site (sandwich) immunoassay for detecting CKMB in samples of biological fluids, such as, human serum. In particular, the assay employs an antibody to CKB which is bound to a solid support (the "capture antibody"), and a labelled antibody to CKMB (the "tracer antibody").

The capture antibody can be a polyclonal antibody obtained from the serum of an animal which has been immunized to CKBB or a monoclonal antibody produced by a hybrid cell line. Monoclonal antibodies and, in particular, mouse monoclonal antibodies of the IgG1 type, are preferred. Polyclonal antibodies to CKBB are available from, among others, Pel Freez Corporation and Ventrex Corporation; a monoclonal antibody to this antigen is available from Hybritech, Inc. As demonstrated in Example 2 below, anti-CKB is the preferred capture antibody since the assay is essentially completely free from both CKMM and CKBB interference when this capture antibody is used in combination with a CKMB tracer antibody even when only a single incubation step and a single separation/wash step are used.

The capture antibody can be carried by a variety of solid supports, including, for example, glass and plastic beads, magnetic particles, the outer surface of a rod, disk, or other structure, the inner surface of a test tube or other container, and the like. Similarly, various techniques known in the art can be used to attach the capture antibody to its solid support. Because of their ease of use, magnetic particles and, in particular, magnetic particles of the type disclosed in U.S. Patent No. 4,554,088, are preferred for use with the present invention. Capture antibodies can be conveniently coupled to particles of this type by coating the particles with silane, activating the particles with glutaraldehyde, washing the particles, and then adding the antibody. See Reichlin, M., "Use of Glutaraldehyde as a Coupling Agent for Proteins and Peptides", Method of Enzymology, 70:159-165, (1980).

The tracer antibody is a monoclonal antibody to CKMB. As discussed above, in terms of providing an antibody suitable for use in a commercial assay, mouse monoclonal antibodies of the IgG1 type are preferred. Antibodies of this type are more stable and easier to label than, for example, IgG2b type antibodies. In particular, it has been found that use of an IgG1 tracer antibody in the present assay results in more signal than that generated under the same conditions by an IgG2b antibody. Also, IgG1 type antibodies can be frozen and thawed without suffering significant losses in activity. In a commercial setting, such freezing and thawing can occur, for example, during shipping and storage of assay kits and components.

As discussed in detail in Example 1 below, mouse monoclonal antibodies to CKMB of the IgG1 type can be prepared by immunizing mice with purified CKMB, removing splenocytes from the immunized mice, fusing the splenocytes with a mouse immortal cell line, and screening the resulting hybridomas for cells producing antibodies to CKMB. The antibodies produced by the selected hybridomas are then further screened for antibody type and cross-reactivity with CKBB and CKMM. Those of the IgG1 type having low cross reactivities, e.g., less than 1% for both CKBB and CKMM, constitute the preferred tracer antibodies for use with the present invention.

The anti-CKMB antibody can be labelled with a variety of labels, including, for example, radioactive labels, fluorescent labels, enzymatic labels, chemiluminescent labels, and the like. Chemiluminescent labels and, in particular, chemiluminescent labels employing an acridinium ester as the chemiluminescent material, are preferred.

As demonstrated in Example 3 below, by using a label of this type, concentrations of CKMB in serum samples can be determined by the ultimate user through the use of just two calibrator samples, as opposed to four or more standards as are often required with other labels. A description of the preferred labels an suitable techniques for coupling the labels t antibodies can be found in EP-A-263657.

In its preferred embodiments, the complete assay requires the performance of only four steps: 1) incubation, 2) separation, 3) washing, and 4) counting. Additional incubation and wash steps, of course, can be used in the practice of the invention, if desired.

As demonstrated below in Example 3, incubations at room temperature as short as 30 minutes or even as short as 10 minutes when the assay is performed in a "stat" mode, can be used. Also, through the use of an acridinium ester label, the user only needs to run two calibrator samples to convert the measured light output for a patient sample to a concentration value. In addition, through the use of magnetic particles as the solid support, separations are rapidly and easily performed.

Through the combined effects of each of these factors, the overall assay of the present invention is both easy to use and quick to perform. In comparison to prior art methods where it typically took on the order of two hours or more to obtain a reading for a sample, the present assay can be easily completed in less than an hour. In view of the criticality of being able to diagnose myocardial infarctions quickly, the short overall assay time and ease of performance of the present invention are important measures of its value.

Based on the foregoing description, the invention will now be further illustrated by the following specific examples.

Example 1

Preparation of Anti-CKMB Monoclonal Antibodies

Monoclonal antibodies to human CKMB were prepared using the following immunization, fusion, screening, and isotype characterization procedures.

Immunization Protocol: Six-week old female A/J mice, H-2a haplotype, (Jackson Laboratories, Bar Harbor, ME 04609), were injected intraperitoneally with 30 $\mu$g of purified CKMB (Scripps Laboratories, San Diego, CA) emulsified in an equal volume of complete Freund's adjuvant (CFA) (Difco Laboratories, Detroit, Michigan) on day 0 and day 21. One month later the same amount of antigen in incomplete Freund's adjuvant was administered in the foot pads and subcutaneously. Five weeks later, 20 $\mu$g in CFA was injected intraperitoneally. The final boost of 19 $\mu$g in sterile saline was administered intravenously four days prior to the fusion and seven weeks after the last injection.

Fusion Protocol: Splenocytes from an immunized mouse ($1 \times 10^8$) were fused with cells ($2 \times 10^7$) from a Balb/c mouse myeloma cell line (Sp2/0-Ag14 cells available from the American Type Culture Collection, Rockville, Maryland under accession number ATCC CRL-1581), in the presence of polyethylene glycol (avg. mol. wt. 950-1050) (J.T.Baker Company, Phillipsburg, N.J. 05865), essentially following the published procedure of Kohler and Milstein (Nature, 256:495-497, (1975)). The fusion was distributed to wells in 96-well microtiter tissue culture plates (Corning Glass Works, Corning NY) and incubated at 37°C in a 5% $CO_2$ atmosphere. After two weeks, the hybridomas were screened for antibodies reactive with CKMB.

Screening Procedure: The hybridomas were screened for antibodies against CKMB using a double antibody chemiluminescent assay. Purified CKMB was labeled with dimethyl acridinium ester (n-hydroxysuccinimide) (DMAE-NHS) as follows. To 100 $\mu$g purified CKMB in 1 ml labeling buffer (0.1 M sodium phosphate, 0.15 M sodium chloride, pH 8.0) were added 5 $\mu$g DMAE-NHS in 0.1 ml dimethylformamide. The mixture was vortexed and incubated for fifteen minutes at room temperature. The mixture was then applied to a Sephadex (Registered Trade Mark) G-25 (PD-10) column (Pharmacia Inc., Piscataway, NJ 08854) and the void volume collected. The labeled CKMB was diluted in 0.1% bovine serum albumin (BSA) and phosphate buffered saline pH 7.4 with 0.01% thimerosal and stored at 4°C.

The screening procedure consisted of combining 100 $\mu$l of CKMB-DMAE with 100 $\mu$l of hybridoma culture supernatant in 12x75 mm polystyrene tubes (Walter Sarstedt, Princeton, NJ 08540) and incubating the solution for 2 hours at room temperature. A second antibody, i.e., goat anti-mouse Ig (Cambridge Medical Diagnostics, Inc., Billerica, MA 01865), immobilized on magnetic particles (Advanced Magnetics, Inc., Cambridge, Massachusetts; see Groman et al., "Product Application Focus, BioMag -- A New Support for Magnetic Affinity Chromatograph", Biotechniques, March/April 1985, pages 156-160), was added to the reaction tubes and incubated for 15 minutes at room temperature.

A Corning magnetic separation unit (Ciba-Corning Diagnostics, Walpole, MA 02135) was used to separate the antigen/double-antibody complexes from the rest of the solution. The complexes were washed one time with distilled water and counted using a photon counter (810 Luminometer, Ciba-Corning Diagnostics, Walpole, MA 02135). See Weeks et al., "Chemiluminescence Immunoassay", J. of Clin. Immunoassay, 7:82-88, (1984).

The hybridoma supernatants were considered positive if the counts were at least three times the negative control supernatants which were from growth negative wells. The positive hybridomas were selected and transferred to a 24-well tissue culture plate (Costar, Cambridge, MA 02139) where the cell lines were allowed to expand. The culture supernatant was harvested from the 24-well plates and retested for antibody to CKMB.

Isotype Characterization Procedure: The isotypes of the monoclonal antibodies were determined using the Mono-AB ID Enzyme Immunoassay Kit (Zymed Laboratories, San Francisco, CA 94080).

Following the foregoing procedures, two anti-CKMB monoclonal antibodies potentially suitable for use in the assays of the present invention, were identified (antibodies "007" and "13G1"). Both antibodies were of the IgG1 type and had kappa light chains. In addition to producing anti-CKMB antibodies, the fusions were also found to have produced anti-CKM and anti-CKB antibodies.

The hybridomas which produced the 007 and 13G1 antibodies were cloned by limiting dilution to ensure a monoclonal antibody cell line that was stable. See Goding, supra, at page 85. Both cell lines were found to be stable.

For purposes of further evaluation, the antibodies produced by the cell lines were purified as follows. 50 ml of culture supernatant were concentrated using an Amicon Concentrator (Amicon Corporation, Danvers, MA). The concentrate was then purified using an AFFi-Gel (Registered Trade Mark) Bio-Rad Protein A MAPS II KIT (Bio-Rad Laboratories, Richmond, CA 94801). Finally, the antibodies were dialyzed against 0.1 M sodium phosphate, 0.15 M sodium chloride, pH 8.0. Purity of the antibodies was confirmed by performing high resolution gel electrophoresis.

In order to assess the suitability of the two anti-CKMB monoclonals for two-site immunoassays, standard curves for varying concentrations of CKMB, CKBB, and CKMM were constructed using the potential anti-CKMB antibodies and anti-CKM and anti-CKB monoclonal antibodies (antibodies "AH6" and "CA8", respectively), which had been produced by procedures similar to those used to produce the anti-CKMB antibodies, but with CKMM and CKBB used as the antigens. In particular, the standard curves were constructed by labeling the tracer antibody with DMAE and coupling the capture antibody to magnetic particles (Advanced Magnetics, supra).

The labeling of the tracer antibodies was performed as follows. To 0.25 mg antibody in 1 ml of labelling buffer (0.1 M sodium phosphate, 0.15 M sodium chloride, pH 8.0) was added 40 micrograms DMAE-NHS in 0.2 ml dimethylformamide. After 15 minutes at room temperature, 5 mg DL-lysine in 0.5 ml labelling buffer was added to stop the reaction. The reaction mixture was applied to a small Sephadex G-25 column (PD-10), and the void volume was collected. The buffer system used for chromatography and storage of tracers was "tracer buffer", which consisted of 0.01 M sodium phosphate, 0.1 M sodium chloride, 0.25% BSA, 0.1% sodium azide, pH 7.4. Further purification of the tracer was achieved by chromatography on Sephacryl (Registered Trade Mark) S-400. The tracer in 3 ml was applied to a Sephacryl S-400 column and chromatographed in "tracer buffer". Column sizes of 10 ml, 20 ml, and 200 ml were tested. The large column separated the tracer from larger aggregates and minor peaks that eluted after the tracer peak. The small columns exhibited one main peak. In both cases, however, a 10-fold increase in signal/noise ratio was found due to decrease in background. It is believed that the Sephacryl column removed unidentified contaminants by adsorption.

The assay format employed to prepare the standard curves was as follows (the format varied somewhat from experiment to experiment, but not to an extent which would affect the final results): 0.1 ml standard was added to 0.1 ml tracer antibody and vortexed. Depending on the specific experiment, 0.1, 0.2, or 0.5 ml of capture antibody was added, and the mixture was incubated for 10 minutes, 30 minutes, or 1 hour at room temperature, again depending on the specific experiment. The complex was separated using a Corning magnetic separation unit and washed with distilled water. The assay was counted on an 810 Luminometer.

The results are shown in Figure 1. As shown therein, monoclonals 007 and 13G1 exhibited CKMB "sandwich" compatibility with both anti-CKM and anti-CKB, but not with each other. The anti-CKM (AH6) and anti-CKB (CA8) antibodies also exhibited CKMB "sandwich" compatibility. The lack of "sandwich" formation with CKMM and CKBB antigens (up to 1000 ug/l), illustrate the high specificities (greater than 99.9%) of the 007, AH6 and CA8 antibodies. The 13G1 antibody was slightly cross reactive with CKBB. Accordingly, although this antibody was considered suitable for use in a CKMB assay, it was considered less preferred than the 007 antibody.

The 007 antibody was further characterized in terms of its specificity. Specifically, binding of CKMB-DMAE to this antibody was determined in the presence of up to 10 ug/ml of purified CKBB, CKMM, and CKMB (Scripps Laboratories, San Diego, CA). The isoenzyme standards were prepared from stock solutions stored in 50% glycerol at -20°C. Each isoenzyme was diluted to the desired concentrations in 50 mM PIPES, 100 mM sodium chloride, 5% BSA, 0.1% sodium azide, 1 mM EDTA, 1 mM dithiothreitol (pH 6.7).

To 100 $\mu$l of a limiting dilution of culture supernatant, i.e., the dilution which gave 50% of maximum binding, was added 100 $\mu$l CKMB-DMAE and 100 $\mu$l isoenzyme standard. The solution was incubated for 2 hours at room temperature. Goat anti-mouse Ig magnetic particles (Advanced Magnetics, supra) were added and incubated for 15 minutes at room temperature. The mixture was separated and washed as described above in connection with the screening procedure. Inhibition of counts indicated reactivity of the monoclonal antibody with the isoenzyme.

The results are shown in Figure 2. As shown therein, the 007 antibody exhibited essentially no cross reactivity with CKMM or CKBB up to concentrations of 10 $\mu$g/ml.

The hybrid cell line which produces the 007 anti-CKMB antibody has been deposited with the American Type Culture Collection, Rockville, Maryland, and has been assigned accession number HB 9389. The hybrid cell line which produces the CA8 anti-CKB antibody has also been deposited with the ATCC and has been assigned accession number HB 9388. As with the 007 antibody, the CA8 antibody is of the IgG1 type and has kappa light chains. Similarly, the cell line which produces the CA8 antibody is a stable cell line.

## Example 2

### Assay Format

This example illustrates the development of a CKMB assay configuration which is not susceptible to interferences by CKMM or CKBB, and yet requires only one incubation step.

CKMB standard curves were generated in the presence and absence of either 5000 $\mu$g/l CKMM or 1000 $\mu$g/l CKBB using four different assay configurations, i.e, anti-CKM (AH6) as the capture antibody with either anti-CKB (CA8) or anti-CKMB (007) as the tracer antibody, and anti-CKB (CA8) as the capture antibody with either anti-CKM (AH6) or anti-CKMB (007) as the tracer antibody. The standard curves were generated in the same manner as the standard curves of Example 1.

When the solid-phase antibody was anti-CKM, the standard curve was inhibited by CKBB when labelled anti-CKB was the tracer antibody (Figure 3A), but not when anti-CKMB served as the tracer (Figure 3B). The inhibition by CKBB could be removed by either a wash step prior to adding the labelled anti-CKB, or by a multi-fold increase in the amount of the tracer. Increasing the amount of tracer, however, led to a high non-specific signal. With anti-CKM as the capture antibody, the standard curve was lowered by CKMM regardless of which tracer was used (Figures 3A and 3B).

When the solid-phase antibody was anti-CKB and the tracer antibody was anti-CKM, a strong inhibition of the standard curve by CKMM was seen (Figure 3C). However, no inhibition by CKBB was seen (Figure 3C). A wash step prior to addition of the tracer was effective in eliminating the CKMM inhibition.

Finally, when the solid-phase antibody was anti-CKB and the tracer antibody was anti-CKMB, no interference by either CKMM or CKBB occurred (Figure 3D). Accordingly, this configuration is superior to each of the other configurations, since it both avoids interferences and involves only a single incubation step.

## Example 3

### Assay Optimization and Testing

This example relates to the optimization and testing of the preferred CKMB assay configuration of Example 2 wherein anti-CKB (CA8) and anti-CKMB (007) are used as the capture and tracer antibodies, respectively.

The standard curve generated with the 007 antibody as the tracer antibody was found to be sensitive to medium pH. Specifically, a gradual decrease in signal was observed as the pH was increased from 6.5 to 8.0, with the signal at pH 7.4 being 40% lower than at pH 6.5. Consequently, the reagents used in the assay were buffered with 0.1 M PIPES buffer at pH 6.5, and a medium/sample ratio of 7 was used to prevent potential bias due to serum samples having abnormal pH values.

Several recent publications have described interference by heterophilic, possibly anti-mouse, antibodies, in immunoassays that utilize mouse monoclonal antibodies. See Thompson, R. J., et al.,

"Circulating Antibodies to Mouse Monoclonal Immunoglobulins in Normal Subjects -- Incidence, Species Specificity, and Effects of a Two-Site Assay for Creatine-Kinase-MB Isoenzyme", Clin. Chem., 32:476-481, (1986); and Murthy, V. V., et al., "Activity Concentration and Mass Concentration (Monoclonal Antibody Immunoenzymometric Method) Compared for Creatine Kinase MB Isoenzyme in Serum", Clin. Chem., 32:1956-1959, (1986). Therefore, 0.1% (v/v) non-immune mouse serum was added to the assay medium, with no apparent effect on the standard curve.

Using the foregoing reagents, the following assay protocol was employed in comparing the assay of the present invention with existing CKMB assays:

1. 100 $\mu$l of sample, control, standard, or calibrator are added to a test tube. (Samples having CKMB concentrations greater than 500 $\mu$g/l are first diluted. The preferred diluent is the low calibrator used to convert photon measurements to concentration values. See discussion below.)

2. 100 $\mu$l of anti-CKMB tracer antibody are then added to the tube.

3. The tube is vortexed 3 times for 5 seconds, with foaming being minimized.

4. 500 $\mu$l of anti-CKB capture antibody coupled to magnetic particles (Advanced Magnetics) are added to each tube.

5. The tube is vortexed 3 times for 5 seconds, with foaming being minimized.

6. The tube is incubated for 30 minutes +/-1 minute at room temperature (between 15 and 30°C).

7. The solid phase is separated in a Corning magnetic separation unit for 3 minutes.

8. The tube is decanted and drained for 3 minutes and then blotted.

9. 1.0 ml distilled or deionized water is added to the tube.

10. The tube is vortexed 3 times for 5 seconds, with foaming being minimized.

11. The solid phase is separated in the magnetic separation unit for 3 minutes.

12. The tube is decanted and drained for 3 minutes, and then blotted.

13. 100 $\mu$l distilled water is added to the tube.

14. The tube is vortexed 3 times for 5 seconds, with foaming being minimized.

15. The photon output of the tube is measured for 2.0 seconds using a Magic Lite (Registered Trade Mark) Analyzer sold by Ciba Corning Diagnostics (Walpole, Massachusetts).

Conversation of photon output to concentration values is performed using two calibrators, i.e., a high calibrator and a low calibrator. Specifically, a full standard curve is obtained for each lot of reagents by performing the above procedure on a series of standards having known amounts of CKMB. This standard curve is supplied to the user, and the user programs the standard curve into his Magic Lite Analyzer. The high and low calibrators are used by the analyzer to adjust the standard curve for the specific conditions which the user employed in performing the assay.

Expected values for the assay were determined by performing the assay on 621 diagnosed patient samples in the following categories: non-hospitalized normals (228), diagnosed MI patients (180), and hospitalized non-cardiac related patients (213). Based on this study, a normal range of 0 to 7 $\mu$g/l for non-hospitalized normals and of 0 to 10 $\mu$g/l for hospitalized non-cardiac related normals were found to give the best diagnostic efficiency. Based on the study, it was also concluded that a peak serum CKMB of greater than 10 $\mu$g/l can generally be said to indicate myocardial injury. However, as recognized in the art, elevated serum CKMB concentrations of this magnitude can arise from cardiac conditions other than MI (e.g., congestive heart failure, myocarditis and ischemia) or from non-cardiac tissues (e.g., skeletal muscle). See Lott et al., "Serum Enzymes and Isoenzymes in the Diagnosis and Differential Diagnosis of Myocardial Ischemia and Necrosis", Clin. Chem., 26:1241, 1980.

In addition to the foregoing "standard" assay protocol, a "stat" assay protocol was also developed. This protocol was identical to the standard protocol except that the incubation time was decreased from 30 minutes to 10 minutes (+/- 30 seconds). The correlation of the stat assay with the standard assay was found to be r = 0.998 with a regression equation of: stat = 0.96 standard - 3.5 $\mu$g/l.

A typical standard curve for the standard assay is shown in Figure 4. The standards ranged from 1-700 $\mu$g/l CKMB, with maximal tracer binding of 25%. No "hook effect" was noted up to 1000 $\mu$g/l CKMB, and up to 120 assay tubes could be run at a time without "end of run" effect.

The minimal detectable dose for the standard assay was 0.65 $\mu$g/l, and the non-specific binding was 0.07% of the added label. In comparing the binding kinetics of the capture and tracer antibodies, it was observed that after 10 minutes incubation more than 90% of CKMB had been bound to the solid-phase. The tracer, however, had not yet reached equilibrium. Consequently, for an assay incubation time of 10 minutes, i.e., the stat assay, the standard curve was about 50% lower than that for 30 minutes incubation. Under these conditions the minimal detectable dose was 1 $\mu$g/l.

The accuracy and precision of the standard assay were tested as follows. 146 serum samples, in the range of 1.14 to 263 $\mu$g/l, were assayed

using the method of the present invention and using a conventional electrophoresis technique. The assay of the present invention correlated well with the electrophoresis method (r = 0.938), with a linear regression equation of: CKMB (present invention) = 1.03 CKMB (electrophoresis) - 0.719 $\mu$g/l (see Figure 5).

Similarly, for 168 samples ranging from 0.1 to 340 $\mu$g/l, the standard assay correlated well with the Tandem-E CKMB immunoenzymatic assay sold by Hybritech, Inc., (r = 0.942), the regression equation being: CKMB (present invention) = 1.16 CKMB (Tandem-E) + 17.5 $\mu$g/l.

To further assess the accuracy of the assay, three serum samples (1.7 - 105 $\mu$g/l) were diluted 1:2 and 1:4 with the lowest standard and assayed for CKMB. Recoveries ranged from 81% to 110% with a mean of 93%.

Several samples with macro-CK-1 gave accurate results. Similarly, hemolysis, lipemia, and bilirubin in concentrations up to 30 mg/dl were found to have insignificant effects on the assay.

The intra-assay CV values, obtained from 3 samples which were assayed 3-5 times in each of 26 assays (N = 96), were 12.7%. 8.1%, and 4.1% for patient sera containing 2.5, 4.1 and 210 $\mu$g/l of CKMB, respectively. For the same samples, the inter-assay CV values were 14.4%, 11.7% and 6.8%

With regard to stability of the reagents, it was found that both the anti-CKB antibody bound to magnetic particles and the anti-CKMB antibody labelled with DMAE were stable for at least six months, when stored in an aqueous medium at 4°C. Significantly, with regard to the Vaidya et al. method discussed above, it was observed that the enzymatic activity of CKMB standards was reduced by 40% when stored at 25°C in an aqueous medium for one day. The CKMB mass of the same standards, on the other hand, was found to be completely stable when stored under the same conditions for two days.

As demonstrated by the foregoing, by labelling anti-CKMB with acridinium ester and immobilizing anti-CKB on magnetic particles, the present invention provides a rapid and highly sensitive assay for CKMB. The assay achieves a minimal detectable dose of at least 1 $\mu$g/l CKMB for incubations as short as 10 minutes at room temperature, and the standard curve is effective up to 700 $\mu$g/l CKMB. When applied to serum samples, the assay correlates well with existing methods and does not suffer from interference due to the presence of macro-CK-1 or high levels of CKMM and CKBB in patient samples. In sum, the assay of the invention is faster, simpler to use, and less subject to interference than prior art assays and yet maintains the sensitivity of those assays.

## Claims

1. A method for detecting the MB isoenzyme of creatine kinase in a biological fluid characterised in that it comprises:

   (a) forming a mixture of (i) a sample of the biological fluid, (ii) an antibody to the B monomer of creatine kinase, the said antibody being bound to a solid support, and (iii) a labelled, mouse monoclonal antibody of the IgG1 type specific to the MB isoenzyme of creatine kinase;

   (b) incubating the mixture;

   (c) separating the solid support from the mixture; and

   (d) detecting the amount of label associated with the solid support.

   (a) - (d) being completed in about 1 hour.

2. A method as claimed in claim 1 wherein the antibody to the MB isoenzyme of creatine kinase is labelled with a chemiluminescent material.

3. A method as claimed in claim 2 wherein the chemiluminescent material is an acridinium ester, preferably dimethyl-acridinium ester.

4. A method as claimed in any of claims 1 to 3 wherein the solid support comprises magnetic particles.

5. A method as claimed in any of claims 1 to 4 wherein it additionally comprises determining the concentration of the MB isoenzyme of creatine kinase in the sample by generating a standard curve by performing (a) - (d) on samples having known concentrations of the MB isoenzyme and using the standard curve to convert the detected amount of label into a concentration value.

6. A monoclonal antibody characterised in that it is produced by a murine-derived hybrid cell line, the said antibody being capable of specifically binding to at least one antigenic determinant of the MB isoenzyme of human creatine kinase and being of the IgG1 type and not being substantially cross-reactive with the BB and MM isoenzymes of human creative kinase.

7. A monoclonal antibody as claimed in claim 6 wherein the murine-derived hybrid cell line is ATCC HB 9389 or HB 9388.

8. A murine-derived hybrid cell line ATCC HB 9389 or HB 9388.

9. A mutant of a hybrid cell line as claimed in claim 8 characterised in that it produces an antibody which is capable of specifically binding to at least one antigenic determinant of the MB isoenzyme of human creatine kinase and is of the IgG1 type.

**Revendications**

1. Un procédé pour détecter l'isozyme MB de la créatine-kinase dans un liquide biologique, caractérisé en ce qu'il consiste à :

(a) former un mélange de (i) un échantillon du liquide biologique, (ii) un anticorps dirigé contre le monomère B de la créatine-kinase, ledit anticorps étant lié à un support solide, et (iii) un anticorps monoclonal de souris, marqué, du type IgG1 spécifique envers l'isozyme MB de la créatine-kinase ;

(b) faire incuber le mélange ;

(c) séparer le support solide du mélange ; et

(d) détecter la quantité de marqueur associé au support solide,

(a) à (d) étant achevés en 1 heure environ.

2. Un procédé tel que revendiqué dans la revendication 1, dans lequel l'anticorps dirigé contre l'isozyme MB de la créatine-kinase est marqué par une substance chimioluminescente.

3. Un procédé tel que revendiqué dans la revendication 2, dans lequel la substance chimioluminescente est un ester d'acridinium, de préférence un ester de diméthyl-acridinium.

4. Un procédé tel que revendiqué dans l'une quelconque des revendications 1 à 3, dans lequel le support solide comprend des particules magnétiques.

5. Un procédé tel que revendiqué dans l'une quelconque des revendications 1 à 4, dans lequel le procédé consiste de plus à déterminer la concentration de l'isozyme MB de la créatine-kinase dans l'échantillon en établissant une courbe d'étalonnage par exécution de (a) à (d) sur des échantillons ayant des concentrations connues de l'isozyme MB et en utilisant la courbe d'étalonnage pour convertir la quantité détectée de marqueur en une valeur de concentration.

6. Un anticorps monoclonal, caractérisé en ce qu'il est produit par une lignée cellulaire hybride dérivée de souris, ledit anticorps étant capable de se lier spécifiquement à au moins un déterminant antigénique de l'isozyme MB de la créatine-kinase humaine et étant du type IgG1 et ne présentant sensiblement pas de réaction croisée avec les isozymes BB et MM de la créatine-kinase humaine.

7. Un anticorps monoclonal tel que revendiqué dans la revendication 6, dans lequel la lignée cellulaire hybride dérivée de souris est ATCC HB 9389 ou HB 9388.

8. Une lignée cellulaire hybride dérivée de souris ATCC HB 9389 ou HB 9388.

9. Un mutant d'une lignée cellulaire hybride telle que revendiquée dans la revendication 8, caractérisé en ce qu'il produit un anticorps qui est capable de se lier spécifiquement à au moins un déterminant antigénique de l'isozyme MB de la créatine-kinase humaine et qui est du type IgG1.

**Patentansprüche**

1. Verfahren zum Nachweis des MB-Isoenzyms der Kreatinkinase in einer biologischen Flüssigkeit,

**dadurch gekennzeichnet,**

daß es umfaßt :

(a) Herstellen einer Mischung aus (i) einer Probe der biologischen Flüssigkeit, (ii) einem Antikörper gegen das B-Monomer der Kreatinkinase, wobei dieser Antikörper an einen festen Träger gebunden vorliegt, und (iii) einem markierten monoklonalen Antikörper der Maus vom IgG1-Typ, der spezifisch für das MB-Isoenzym der Kreatinkinase ist;

(b) Inkubieren der Mischung;

(c) Abtrennen des festen Trägers von der Mischung und

(d) Nachweis der Menge an Markierung, die mit dem festen Träger verbunden ist.

(a)-(d) Sind in etwa einer Stunde abgeschlossen.

2. Verfahren nach Anspruch 1, wobei der Antikörper gegen das MB-Isoenzym der Kreatinkinase mit einem chemilumineszierenden Material markiert ist.

3. Verfahren nach Anspruch 2, wobei das chemilumineszierende Material ein Acridiniumester, bevorzugt ein Dimethylacridiniumester ist.

4. Verfahren nach einem der Ansprüche 1 bis 3, wobei der feste Träger magnetische Teilchen enthält.

5. Verfahren nach einem der Ansprüche 1 bis 4, wobei dieses Verfahren zusätzlich umfaßt das Bestimmen der Konzentration des MB-Isoenzyms der Kreatinkinase in der Probe durch Erstellen einer Standardkurve mittels Durchführung der Verfahrensschritte (a) bis (d) bei Proben mit bekannten Konzentrationen an MB-Isoenzym und Verwenden der Standardkurve, um die nachgewiesene Markierungsmenge in einen Konzentrationswert umzurechnen.

6. Monoklonaler Antikörper,
**dadurch gekennzeichnet,**
daß er produziert wird von einer von einer Maus abstammenden Hybridzellinie, wobei dieser Antikörper in der Lage ist, spezifisch an wenigstens eine antigene Determinante des MB-Isoenzyms der menschlichen Kreatinkinase zu binden und vom IgG1 Typ ist und im wesentlichen mit den BB- und MM-Isoenzymen der menschlichen Kreatinkinase nicht kreuzreagiert.

7. Monoklonaler Antikörper nach Anspruch 6, wobei die von einer Maus abstammende Hybridzellinie ATCC HB 9389 oder HB 9388 ist.

8. Von der Maus abstammende Hybridzellinie ATCC HB 9389 oder HB 9388.

9. Mutante einer Hybridzellinie nach Anspruch 8, dadurch gekennzeichnet, daß sie einen Antikörper produziert, der in der Lage ist, spezifisch an wenigstens eine antigene Determinante des MB-Isoenzyms der menschlichen Kreatinkinase zu binden und er vom IgG1 Typ ist.

| Paramagnetic-Particles Immobilized Antibodies | Anti-CK-M (AH6) | | | Anti-CK-B (CA8) | | | Anti-CK-MB (13G1) | | | Anti-CK-MB (007) | | |
|---|---|---|---|---|---|---|---|---|---|---|---|---|
| | CKMM | CKMB | CKBB | CKMM | CKMB | CKBB | CKMM | CKMB | CKBB | CKMM | CKMB | CKBB |
| Anti-Ck-M (AH6) | − | − | − | − | + | − | − | + | − | − | + | − |
| Anti-CK-B (CA8) | − | + | − | − | − | − | − | + | Trace | − | + | − |
| Anti-CK-MB (13G1) | − | + | − | − | + | Trace | | | | − | − | − |

FIG.I

EP 0 288 179 B1

FIG.2

Competitor Added (ug/ml)

% B/B₀

EP 0 288 179 B1

FIG.3A          FIG.3B

EP 0 288 179 B1

FIG.3C

FIG.3D

EP 0 288 179 B1

FIG.4

EP 0 288 179 B1

FIG.5